# EUROPÄISCHE PATENTSCHRIFT

(11) **EP 1 522 587 B1**
(45) Veröffentlichungstag und Bekanntmachung des Hinweises auf die Patenterteilung: **02.04.2008**
(21) Anmeldenummer: 04024956.7
(22) Anmeldetag: 23.04.2002
(51) Int. Cl.: C12N 15/82, H03K 3/015

(54) **Verfahren zur Einbringung von Nukleinsäuren und anderen biologisch aktiven Molekülen in den Kern höherer eukaryontischer Zellen mit Hilfe elektrischen Stroms**
Method for introducing nucleic acids and other biologically active molecules into the nucleus of higher eukaryotic cells by means of an electric current
Procédé permittant d'introduire des acides nucléiques et d'autres molécules actives d'un point de vue biologique, dans le noyau de cellules eucaryotes supérieures, grace à un courant electrique

(30) Priorität: 23.04.2001 DE 10119901
(43) Veröffentlichungstag der Anmeldung: 13.04.2005
(62) Teilanmeldung aus: 02740285.8
(73) Patentinhaber: Amaxa AG, 50829 Köln (DE)
(72) Erfinder: Müller-Hartmann, Herbert, 50937 Köln (DE); Riemen, Gudula, 40764 Langenfeld (DE); Rothmann-Cosic, Kirsten, 10117 Berlin (DE); Thiel, Corinna, 50823 Köln (DE); Altrogge, Ludger, 53894 Mechernich (DE); Weigel, Meike, 50823 Köln (DE); Christine, Rainer, 50678 Köln (DE); Lorbach, Elke, 50935 Köln (DE); Helfrich, Juliane, 08371 Glauchau (DE); Wessendorf, Heike, San Francisco, CA 94107 (US); Siebenkotten, Gregor, 50226 Frechen-Königsdorf (DE)

(56) Entgegenhaltungen:
- US-A- 6 040 184
- US-A- 6 103 084
- US-A- 6 150 148
- LEOPOLD R A ET AL: "Using electroporation and a slot cuvette to deliver plasmid DNA to insect embryos" GENETIC ANALYSIS: BIOMOLECULAR ENGINEERING, ELSEVIER SCIENCE PUBLISHING, US, Bd. 12, Nr. 5, 1. März 1996 (1996-03-01), Seiten 197-200, XP004052075 ISSN: 1050-3862

## Beschreibung

Die Erfindung betrifft ein Verfahren zur Einbringung von Nukleinsäuren, Peptiden, Proteinen und/oder anderen biologisch aktiven Molekülen in den Zellkern eukaryontischer Zellen mittels elektrischen Stroms, wobei zumindest ein Spannungsimpuls auf die Zellen übertragen wird.

### Hintergrund

Da der Wirkungsort von eukaryontischer DNA der Zellkern ist, muss von außen zugegebene DNA in den Kern gelangen, um abgelesen zu werden. Herkömmliche Transfektionsmethoden bewirken nur einen Transport von DNA durch die Zellmembran ins Zytoplasma. Nur weil bei der Zellteilung höherer Eukaryonten die Kernhülle vorübergehend aufgelöst wird, kann die DNA passiv in den Kern gelangen, so dass von ihr kodierte Proteine exprimiert werden können. Nur sehr kleine DNA-Moleküle (Oligonukleotide) können frei durch die Poren der Kernhülle diffundieren. Zur effektiven Transfektion ruhender oder schwach teilungsaktiver Zellen müssen also Bedingungen geschaffen werden, die dazu führen, dass auch größere DNA-Moleküle in ausreichender Menge durch die Kernmembran in den Kern gelangen. Das hier beschriebene Verfahren ermöglicht dies in höheren eukaryontischen Zellen.

### Stand der Technik

Es ist seit langem bekannt, dass man mit Hilfe elektrischen Stroms DNA aus einem Puffer in Zellen einbringen kann. Die bisher beschriebenen Elektroporationsverfahren sind allerdings auf den Transport von DNA ins Zytoplasma höherer eukaryontischer Zellen ausgelegt, so dass die Expression transfizierter DNA von der Auflösung der Kernhülle während der Zellteilung abhängig bleibt. Keines der bisher bekannten Elektroporationsverfahren befasst sich damit, DNA elektrisch gezielt in den Kern höherer eukaryontischer Zellen zu bringen. Ein Verfahren für die Elektrotransfektion, die auf elektrischen Kerntransport optimiert ist, ist daher nicht bekannt.

Das US-Patent 4,750,100 der Firma Bio-Rad Laboratories, Richmond, USA (1986), beschreibt einen bestimmten Geräteaufbau, der durch eine Kondensatorenentladung maximal 3000 V bei maximal 125 A bereitstellen kann.

Das US-Patent 5,869,326 (Genetronics, Inc., San Diego, USA, 1996) beschreibt einen bestimmten Geräteaufbau, durch den mit Hilfe zweier getrennter Stromquellen zwei, drei oder mehrere Impulse erzeugt werden können. Es wird aber nicht beansprucht oder aufgezeigt, dass diese Impulse eine über den Transport von DNA ins Zytoplasma hinausgehende Wirkung haben.

Das US-Patent 6,008,038 und die europäische Patentanmeldung EP 0 866 123 A1 (Eppendorf-Netheler-Hinz GmbH, Hamburg, 1998) beschreiben ein Gerät, mit dem kurze Impulse von 10-500 µs und maximal 1,5 kV erzeugt werden können, geben aber wieder keinerlei Hinweis darauf, dass bestimmte Bedingungen dazu führen könnten, DNA in den Kern zu befördern

Verfahren zur Einbringung von biologisch aktiven Molekülen in den Zellkern eukaryontischer Zellen nach dem ersten Teil des Patentanspruchs 1 sind aus US 6 103 084 A und US 6 150 148 A bekannt.

Keine der bisher bekannten Verfahren ist darauf optimiert, den effektiven Transport von DNA und/ oder anderen biologisch aktiven Molekülen in den Zellkern bei geringer Mortalität der Zellen zu ermöglichen.

Der Erfindung liegt die Aufgabe zugrunde, ein Verfahren zur Verfügung zu stellen, welches den effektiven Transport von DNA und/oder biologisch aktiven Molekülen in den Zellkern bei geringer Zellmortalität ermöglicht.

### Beschreibung der Erfindung

Erfindungsgemäß ist zur Lösung der Aufgabe vorgesehen, dass die durch den Spannungsimpuls abgegebene Ladungsmenge in zumindest einem wählbaren Zeitintervall gemessen wird, wobei ein Vergleich einer voreingestellten Soll-Ladungsmenge mit der abgegebenen Ist-Ladungsmenge durchgeführt wird, und dass bei Erreichen oder Überschreiten der Soll-Ladungsmenge der Spannungsimpuls abgebrochen wird.

In Ausgestaltung der Erfindung ist vorgesehen, dass zunächst ein erster Impuls mit der Spannung U₁ auf die Zellen übertragen wird und dass anschließend ohne Unterbrechung zumindest ein zweiter Impuls mit der Spannung U₂ ebenfalls auf die Zellen übertragen wird.

Neben der Möglichkeit, die abgegebene Ladungsmenge über den aus der Speichereinrichtung abfließenden Strom zu bestimmen, wird alternativ in einem Zeitintervall ein Vergleich der voreingestellten Soll-Ladungsmenge mit der abgegebenen Ist-Ladungsmenge durchgeführt und bei Erreichen oder Überschreiten der Soll-Ladungsmenge der Leistungshalbleiter gesperrt. Hierbei kann je nach verwendeter Impulsform und Anzahl von Impulsen das zur Bestimmung wählbare Zeitintervall individuell vorgegeben werden, um beispielsweise die abgegebene Ladungsmenge während des ersten oder eines jeden weiteren nachfolgenden Impulses zu bestimmen. Die Bestimmung der abgegebenen Ladungsmenge kann durch die Ermittlung der Differenz zwischen ursprünglicher Ladung zumindest einer der Speichereinrichtungen und der restlichen Ladung erfolgen. Hierbei besteht die Möglichkeit, dass entsprechend der verwendeten Impulszahl mehr als eine der mindestens zwei Speichereinrichtungen schaltungstechnisch eingesetzt wird, wobei jeder Speichereinrichtung zumindest ein Hochspannungsnetzteil, eine Kontrolleinrichtung und ein Leistungshalbleiter zur Übertragung der Ladungsmenge auf die Küvette mit der Zellsuspension zugeordnet wird. Für die Übertragung der Impulse ist vorgesehen, dass der erste Leistungshalbleiter einen Impuls von 2 - 10 kV/cm mit einer Dauer von 10 - 100 µs und einer Stromdichte von mindestens 2 A cm⁻² überträgt und der zweite Leistungshalbleiter ohne Unterbrechung einen Impuls mit einer Stromdichte von 2 - 14 A cm⁻² mit einer Dauer von maximal 100 ms überträgt. Das Zeitintervall zur Bestimmung der abgegebenen Ladungsmenge kann demzufolge mit der Abgabe eines ersten und/oder vorzugsweise eines zweiten oder jeden weiteren Impulses festgelegt werden.

Vorzugsweise erfolgt eine Überwachung der abgegebenen Ladungsmenge des zweiten Impulses, wobei die Einschaltdauer (T₂) des zweiten Impulses durch einen Vergleich der Soll-Ladungsmenge mit der bis zum Messzeitpunkt abgegebenen Ist-Ladungsmenge festlegbar ist und mit Erreichen der Soll-Ladungsmenge endet und wobei zur Bestimmung der Ist-Ladungsmenge ein Messzyklus von 1 msec vorgesehen ist, wobei während der Zeit (T₂) die Kondensatorspannung exponentiell abfällt und bei Erreichen der festgelegten Ladungsmenge (Q₂) der Leistungshalbleiter sperrbar ist.

Alternativ besteht die Möglichkeit, dass nach zumindest einem vorbestimmten Zeitintervall nach Auslösen eines ersten und/oder zweiten Impulses der fließende Strom gemessen wird und bei Über- oder Unterschreiten eines Sollwertes die Dauer des Pulses nachregelbar ist, um die abgegebene Ladungsmenge konstant zu halten. In einer weiteren Alternative besteht die Möglichkeit, dass nach zumindest einem vorbestimmten Zeitintervall nach Auslösen eines ersten und/oder zweiten Pulses der fließende Strom gemessen wird und bei Über- oder Unterschreiten eines Sollwertes eine Fehlermeldung erfolgt, um den Benutzer des Geräts einen Warnhinweis zu geben. Weiterhin besteht die Möglichkeit, dass nach zumindest einem vorbestimmten Zeitintervall nach Auslösen eines ersten und/oder zweiten Pulses der fließende Strom gemessen wird und bei Über- oder Unterschreiten des Sollwertes eine Nachregelung an den Sollwert erfolgt.

Zur Festlegung von evtl. notwendigen Konstanten, insbesondere der verwendeten Küvette mit der Zellsuspension, kann vorgesehen sein, dass eine vorherige Messung des Widerstands der Küvette mit der Zellsuspension erfolgt. Vorzugsweise werden die weiteren notwendigen lmpulsparameter manuell vorgewählt oder ggf. durch die Eingabe eines Codes festgelegt. Es besteht daher auch die Möglichkeit abrufbare Daten über einen Kartenleser zu verwenden. Der Kartenleser kann gleichzeitig dazu verwendet werden, den zeitlichen Verlauf der an die Küvette angelegten Spannung bzw. des durch die Küvette fließenden Stromes zu Dokumentationszwecken für ein oder mehrere lmpulsabgabevorgänge auf einer handelsüblichen Speicherkarte zu speichern. Diese Speicherkarte dient bevorzugt gleichzeitig zur Speicherung der einzustellenden lmpulsparameter.

Durch die schaltungstechnische Regelung der Impulsabgabe wird somit in zuverlässiger und vorteilhafter Weise die Übertragung der vorgesehenen Ladungsmenge zumindest für einen Impuls überwacht und eine kontrollierte und probenabhängige Übertragung einer voreingestellten Ladungsmenge sowie eine kontrollierte Überwachung zur Vermeidung einer Schädigung der in der Probe befindlichen Zellen ermöglicht.

Zur weiteren Sicherheit des Benutzers und der verwendeten Proben ist vorgesehen, dass eine Überstromabschaltung für den ersten und jeden weiteren Impuls vorgesehen ist. Die Überstromabschaltung ermöglicht somit jederzeit die Unterbrechung des Hochspannungsimpulses für den Fall, dass voreingestellte Grenzwerte überschritten werden.

Der beschriebene Hochspannungsimpuls von 2 - 10 kV/cm ist dazu geeignet, Bedingungen zu schaffen, die dazu dienen, dass DNA unabhängig von der Zellteilung in den Zellkern gelangen kann. Um Zellschäden gering zu halten, wird dieser Puls auf 10 bis maximal 200 µs, vorzugsweise 10- 50 µs begrenzt. Dies ist ausreichend, um zellteilungsunabhängige Transfektion zu erreichen. Für die Transfektion von Endothelzellen aus der menschlichen Nabelschnurvene beispielsweise stellte sich ein solcher kurzer einzelner Hochspannungspuls als optimal heraus. Ein weiterer ohne Unterbrechung folgender Impuls niedrigerer Feldstärke und niedrigerer Stromstärke bzw. Stromdichte, aber längerer Dauer, hat Einfluss auf die Effizienz der Transfektion. Durch die deutlich niedrigere Stromdichte kann dieser Puls bei geringen Zellschäden deutlich länger anhalten. Je nach Zelltyp und Empfindlichkeit der Zelle gegenüber elektrischen Entladungen ergibt sich eine optimale Stromdichte bzw. Dauer des 2. Pulses. Solche kombinierten Pulse stellten sich beispielsweise für primäre humane dermale Fibroblasten oder Melanozyten oder verschiedene Zellen des menschlichen Blutes als optimal heraus. In Versuchen mit unterschiedlichen Zelllinien und Expressionssystemen konnte Folgendes gezeigt werden: Je höher die Stromdichte des 2. Pulses ist, desto stärker ist sein Einfluss auf die Transfektionsrate, d. h. den Prozentsatz transfizierter Zellen. Je niedriger die Stromdichte ist, desto mehr bewirkt der 2. Puls einen reinen DNA-Transport in durch den 1. Puls schon transfizierte Zellen. Das Expressionsniveau der transfizierten Zellen nimmt mit steigender Pulsdauer zu, aber nicht der Anteil transfizierter Zellen. Um eine genaue zelltypspezifische Steuerung der Transfektionsrate, des Expressionsniveaus und der Zellvitalität zu erhalten, müssen also Pulsdauer und Stromdichte des zweiten Impulses gesteuert werden.

Für eine präzise Kontrolle des tatsächlich auf die Zellsuspension abgegebenen Pulses wird in einer bevorzugten Ausführungsform die abgegebene Ladungsmenge kontrolliert. Um durch eine wählbare Kondensatorspannung der Speichereinheit die Stromstärke bzw. Stromdichte zu kontrollieren, muss der Widerstand der Küvette und der darin enthaltenen Zellsuspension zu Anfang vorgegeben werden. Es zeigte sich, dass sich durch elektrochemische Prozesse der Widerstand der verwendeten Küvetten mit Aluminiumelektroden während des Pulses ändert. Diese Änderung wird durch einen pulsspezifischen vorgegebenen Korrekturwert berücksichtigt. So lassen sich nach U₂ = R x I₂ x K₂ durch eine Kontrolle der Ladung präzise Impulsformen für den 2. Impuls vorherbestimmen, wobei U₂ die Kondensatorenspannung ist, mit der die Speichereinrichtung aufgeladen wird, R der Widerstand der Küvette und der darin enthaltenen Zellsuspension ist, I₂ der Sollstrom und K₂ pulsspezifische Korrekturwert ist.

Der ohmsche Küvettenwiderstand R kann in einer Ausführungsform der Erfindung unmittelbar vor dem Beginn der Impulsabgaben durch Anlegen einer Prüfspannung gemessen und entsprechend bei der Berechnung der Spannung U₂ berücksichtigt werden. Da der vor den Impulsabgaben gemessene Widerstand - vermutlich aufgrund elektrochemischer Vorgänge - größeren Schwankungen unterliegt als der Widerstand während der Impulsabgaben, erweist es sich als vorteilhaft, den Widerstand R zur Berechnung der Kondensatorspannung U₂ als Parameter fest vorzugeben. In einer bevorzugten Ausführungsform der Erfindung wird der Widerstand der Küvette dessen ungeachtet vor Beginn der lmpulsabgaben gemessen, um zu bestimmen, ob dieser innerhalb eines vorgegebenen Widerstandsfensters liegt. Liegt der gemessene Widerstand außerhalb dieses Fensters, so liegt eine Störung vor und die Impulsabgabe wird nicht freigegeben.

Für jeden Zelltyp lassen sich optimale Bedingungen für Transfektionsrate, Transfektionsintensität und Zellvitalität einstellen. In einer bevorzugten Ausführungsform des Verfahrens sind Feldstärke und Dauer des ersten Pulses und Anfangsstromstärke bzw. -stromdichte und empirische Dauer des zweiten Pulses wählbar und optimale Bedingungen für verschiedene Zelltypen über einen Code einfach einzustellen.

Das Verfahren ist in vorteilhafter Weise für die Transfektion ruhender oder teilungsaktiver eukaryontischer Zellen einsetzbar. Ebenso ist das Verfahren für die Transfektion von primären Zellen wie Zellen des menschlichen Bluts, pluripotenter Vorläuferzellen des menschlichen Bluts, primärer menschlicher Fibroblasten, Endothelzellen, Muskeizellen oder Melanozyten geeignet und kann zu analytischen oder diagnostischen Zwecken oder zur Herstellung eines Arzneimittels zur ex-vivo-Gentherapie eingesetzt werden.

Das erfindungsgemäße Verfahren eignet sich darüber hinaus beispielsweise auch für die Elektrofusion, d.h. Verfahren zur Fusion von Zellen, Zellderivaten subzellulären Partikeln und/oder Vesikeln mittels elektrischen Stroms, bei dem beispielsweise die Zellen, Zellderivate, subzellulären Partikel und/oder Vesikel zunächst in zweckmäßiger Dichte in einer wässrigen Lösung suspendiert werden, die Suspension anschließend in eine Küvette überführt wird, und schließlich eine elektrische Spannung an die Elektroden der Küvette angelegt und ein Stromfluss durch die Suspension erzeugt wird. Alternativ können beispielsweise auch adhärente Zellen, Zellderivate, subzelluläre Partikel und/oder Vesikel oder aber auch beispielsweise adhärente Zellen mit suspendierten Zellen, Zellderivaten, subzellulären Partikeln oder Vesikeln fusioniert werden.

Das hier beschriebene Verfahren verwendet sehr hohe Feldstärken von 2 bis 10 kV/cm, die bewirken, dass DNA und/oder andere biologisch aktive Moleküle unabhängig von der Zellteilung in den Kern gelangen können. Diese Feldstärken liegen weit über den für Elektroporation üblichen und weit jenseits der Feldstärken, die für effiziente Öffnung von Poren in der Zellmembran ausreichend sind (durchschnitlich 1 kV/cm laut Lurquin, 1997, Mol. Biotechnol. 7, 5).

Gegenstand der Erfindung ist daher ein Verfahren zur Umsetzung eines Verfahrens zur Einbringung von Nukleinsäuren, Peptiden, Proteinen und/oder anderen biologisch aktiven Molekülen in den Zellkern höherer eukaryontischer Zellen mit Hilfe elektrischen Stroms, wobei das Einbringen in den Kern durch einen Impuls mit dem 2 - 10 fachen Wert der Feldstärke, die für die Porenöffnung in der Zellmembran ausreichend ist, und einer Dauer von mindestens 10 µs und einer Stromdichte von mindestens 2 A cm⁻² erreicht wird.

Das Einbringen von Nukleinsäuren, Peptiden, Proteinen und/oder anderen biologisch aktiven Molekülen in den Zellkern kann dabei durch einen Impuls von 2-10 kV/cm, bevorzugt 3-8 kV/cm erreicht werden, wobei der Impuls maximal 200 µs lang ist.

Das Verfahren ist so ausgelegt, dass auf den ersten Impuls ohne Unterbrechung ein Stromfluss mit einer Stromdichte von 2 A cm⁻² bis maximal 14 A cm⁻², vorzugsweise bis 5 A cm⁻², von 1 ms bis max. 100 ms, vorzugsweise bis 50 ms, Länge folgen kann.

Da das Verfahren eine Transfektion unabhängig von der Zellteilung ermöglicht, können damit neben teilungsaktiven Zellen auch ruhende oder schwach teilungsaktive primäre Zellen transfiziert werden.

In bevorzugten Ausführungsformen der Erfindung werden primäre periphere menschliche Blutzellen sowie pluripotente Vorläuferzellen des menschlichen Blutes transfiziert.

In weiteren bevorzugten Ausführungsformen umfassen die höheren eukaryontischen Zellen primäre menschliche Fibroblasten, Endothelzellen und Melanozyten.

Die mittels des erfindungsgemäßen Verfahrens transfizierten eukaryontischen Zellen können für diagnostische und analytische Zwecke und zur Herstellung eines Arzneimittels zur ex vivo-Gentherapie verwendet werden.

Das erfindungsgemäße Verfahren ermöglicht eine zellteilungsunabhängige Transfektion und damit eine erhebliche Beschleunigung der Transfektionsexperimente. Bei Transfektionsexperimenten mit Expressionsvektoren ist eine Analyse je nach Promotor und exprimiertem Protein schon wenige Stunden nach der Transfektion möglich.

Der Begriff "biologisch aktive Moleküle" bedeutet Peptide, Proteine, Polysaccharide, Lipide oder Kombinationen oder Derivate dieser Moleküle, solange sie in der Zelle eine biologische Aktivität entfalten.

Zur Verwendung in dem erfindungsgemäßen Verfahren eignen sich besonders Elektroporations-Puffer mit hoher lonenstärke und hoher Pufferkapazität.

Zur Einbringung von Nukleinsäuren in den Zellkern eukaryontischer Zellen kann nach folgendem Protokoll vorgegangen werden: 1 x 10⁵ - 1 x 10⁷ Zellen und bis zu 10 µg DNA werden in 100 µl Elektroporations-Puffer in einer Küvette mit 2 mm Elektrodenabstand für 10 min. bei Raumtemperatur inkubiert und dann gemäß den erfindungsgemäßen Bedingungen transfiziert. Sofort danach werden die Zellen mit 400 µl Zellkulturmedium aus der Küvette gespült und für 10 min. bei 37°C inkubiert. Danach werden die Zellen in 37°C warmem Zellkulturmedium ausplattiert.

Geeignete Küvetten sind solche mit einem Elektrodenabstand von 2 mm oder 1 mm, z. B. handelsübliche Küvetten für die Elektroporation von Prokaryonten.

Der Nachweis, dass die Nukleinsäuren tatsächlich unabhängig von der Zellteilung in den Zellkern gelangen, lässt sich dadurch erbringen, dass Zellen analysiert werden, die sich zwischen Transfektion und Analyse nicht geteilt haben. Das geschieht einerseits durch die Transfektion teilungsinaktiver Zellen, wie z.B. Zellen des peripheren menschlichen Blutes und andererseits, bei teilungsaktiven Zellen, durch eine Analyse wenige Stunden nach der Transfektion zu einem Zeitpunkt an dem sich höchstens ein Bruchteil der Zellen geteilt haben kann.

Neben allgemein gebräuchlichen Abkürzungen wurden folgende Abkürzungen verwendet:
- FACS: Flurorescence activated cell sorting
- FCS: foetales Kälberserum
- PBMC: periphere mononukleäre Blutzellen
- PE: Phycoerythrin

### Beispiele

Die nachstehenden Beispiele veranschaulichen die Erfindung, sind jedoch nicht als einschränkend aufzufassen.

### Beispiel 1

### Transfektion von zytotoxischen T-Zellen aus menschlichem Blut

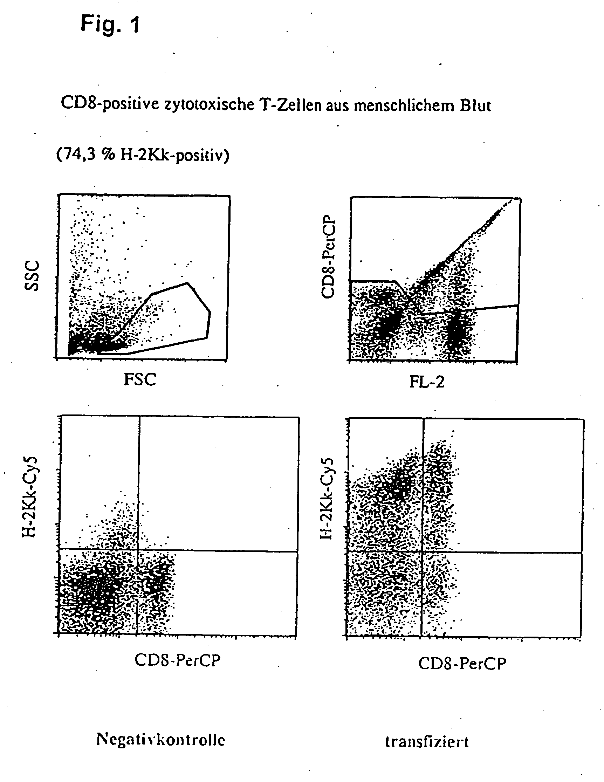

Frisch aufbereitete unstimulierte (teilungsinaktive) mononukleäre Zellen aus peripherem menschlichem Blut (PBMC) wurden mit einem Vektor, der für die schwere Kette des Maus MHC Klasse I Proteins H-2K^{k} kodiert, transfiziert. 5 x 10⁶ Zellen wurden mit 5 µg Vektor-DNA in einem Puffer mit hoher Pufferkapazität (48 mM x pH⁻¹) und hoher lonenstärke (280 mM) bei Raumtemperatur in eine Küvette mit 2 mm Elektrodenabstand gegeben und durch einen Puls von 1000 V und 100 µs Dauer, gefolgt von einem Stromfluss mit einer Stromdichte von 5 A cm⁻² und 40 ms Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 24 h Inkubation wurden die Zellen nacheinander mit Digoxygenin-gekoppeltem anti-H-2K^{k}-Antikörper und Cy5-gekoppeltem anti-Digoxygenin-Antikörper, sowie mit einem PerCP-gekoppeltem anti-CD8-Antikörper zur Identifizierung humaner zytotoxischer T-Zellen inkubiert und mit einem Durchfluß-Zytometer (FACScalibur, Becton Dickinson) analysiert. Durch Färbung mit Propidiumjodid wurde die Anzahl toter Zellen bestimmt. Wie in Figur 1 gezeigt, exprimieren 74,3 % der lebenden Zellen das H-2K^{k}-Antigen, was einer sehr hohen Transfektionseffizienz entspricht.

### Beispiel 2

### Transfektion von humanen hämatopoietischen Stammzellen (CD34)

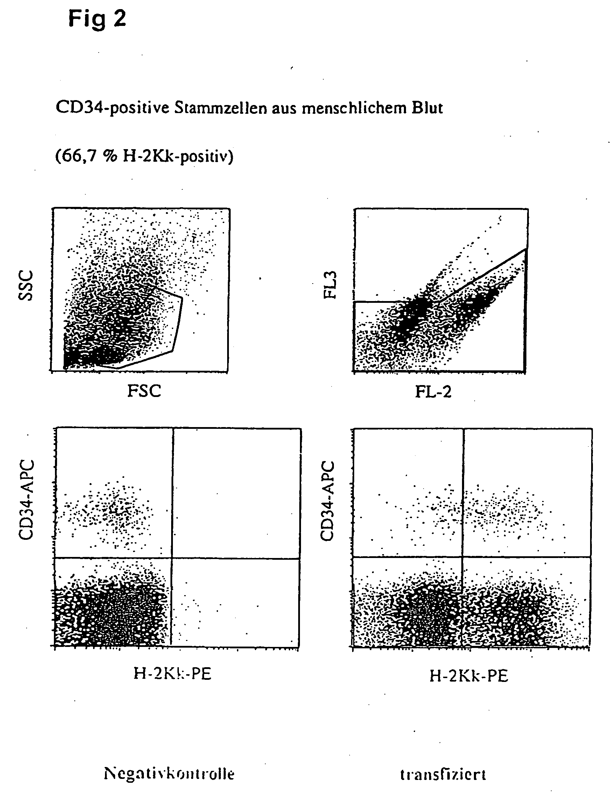

Aus, wie in Beispiel 1 beschrieben, frisch aufbereiteten PBMC wurden CD34-positive Zellen durch magnetische Zellsortierung vorangereichert. Anschließend wurden jeweils 1 x 10⁴ CD34-positive Zellen mit 1 x 10⁶ PBMCs gemischt, mit 5 µg H-2K^{k}-Expressionsvektor-DNA in einem Puffer mit hoher Pufferkapazität (54 mM x pH⁻¹) und hoher Ionenstärke (260 mM) bei Raumtemperatur in eine Küvette mit 2 mm Elektrodenabstand gegeben und durch eine Puls von 1000 V und 100 µs Dauer, gefolgt von einem Stromfluss mit einer Stromdichte von 4 A cm⁻² und 20 ms Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 16 h Inkubation wurden die Zellen nacheinander mit Phycoerythrin-gekoppeltem anti-H-2K^{k}-Antikörper, sowie mit einem APCgekoppeltem anti-CD34-Antikörper zur Identifizierung humaner hämatopoietischer Stammzellen inkubiert und mit einem Durchfluß-Zytometer (FACScalibur, Becton Dickinson) analysiert. Durch Färbung mit Propidiumjodid wurde die Anzahl toter Zellen bestimmt. Wie in Figur 2 gezeigt, exprimieren 66,7 % der Zellen das H-2K^{k}-Antigen, was ebenfalls einer hohen Transfektionseffizienz entspricht.

### Beispiel 3

### Transfektion von humanen neonatalen dermalen Fibroblasten (NHDF-Neo)

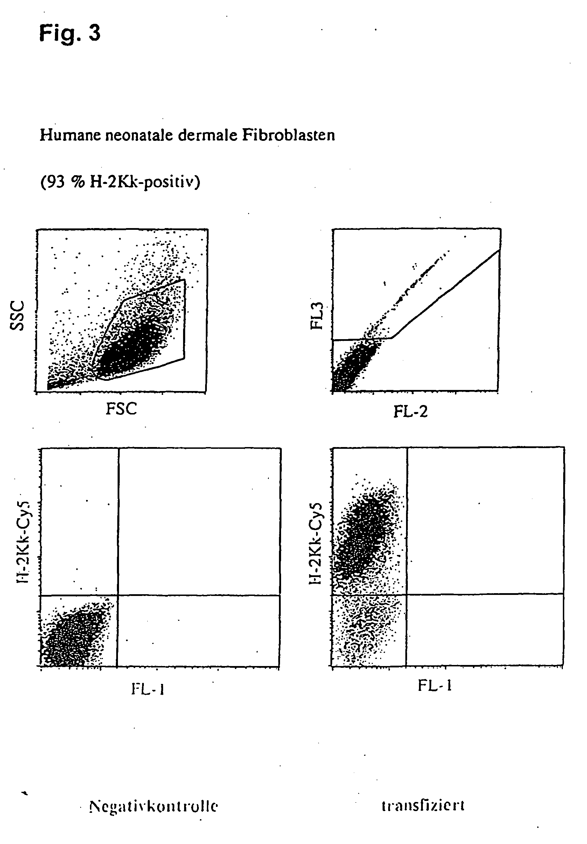

Humane neonatale dermale Fibroblasten (5 x 10⁵ Zellen) wurden mit 5 µg H-2K^{k}-Expressionsvektor-DNA in einem Puffer mit hoher Pufferkapazität (67 mM x pH⁻¹) und hoher Ionenstärke (380 mM) bei Raumtemperatur in eine Küvette mit 2 mm Elektrodenabstand gegeben und durch einen Puls von 1000 V und 100 µs Dauer, gefolgt von einem Stromfluss mit einer Stromdichte von 6A cm⁻² und 33 ms Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 5 h Inkubation wurden die Zellen mit einem Cy5-gekoppeltem anti-H-2K^{k}-Antikörper inkubiert und mit einem Durchfluß-Zytometer (FACScalibur, Becton Dickinson) analysiert. Durch Färbung mit Propidiumjodid wurde die Anzahl toter Zellen bestimmt. Wie in Figur 3 gezeigt, exprimieren 93 % der Zellen das H-2K^{k}-Antigen, was einer sehr hohen Transfektionseffizienz entspricht.

### Beispiel 4

### Transfektion von humanen neonatalen Melanozyten

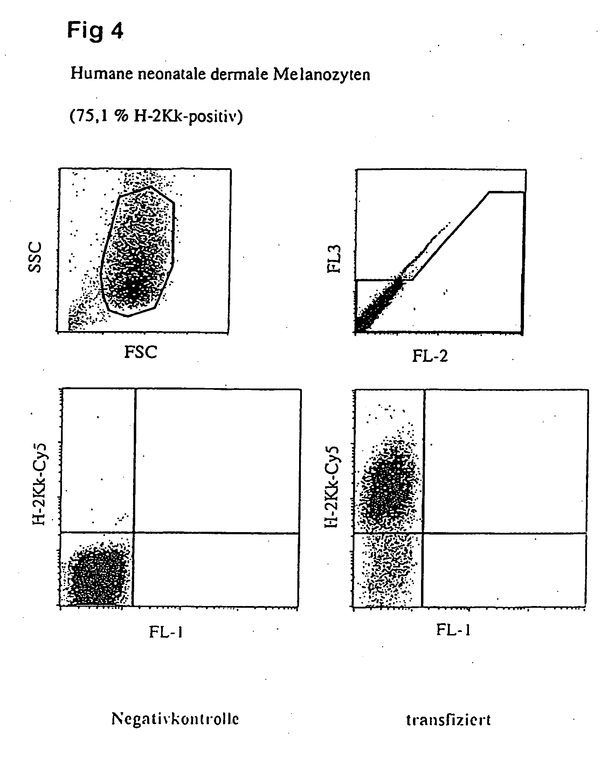

Humane neonatale Melanozyten (2,5 x 10⁵ Zellen) wurden mit 5 µg H-2K^{k}-Expressionsvektor-DNA in einem Puffer mit hoher Pufferkapazität (54 mM x pH⁻¹) und hoher lonenstärke (260 mM) bei Raumtemperatur in eine Küvette mit 2 mm Elektrodenabstand gegeben und durch einen Puls von 1000 V und 100 µs Dauer, gefolgt von einem Stromfluss mit einer Stromdichte von 6 A cm⁻² und 33 ms Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 5 h Inkubation wurden die Zellen mit einem Cy5-gekoppeltem anti-H-2K^{k}-Antikörper und mit einem Durchfluß-Zytometer (FACScalibur, Becton Dickinson) analysiert. Durch Färbung mit Propidiumjodid wurden die Anzahl toter Zellen bestimmt. Wie in Figur 4 gezeigt, exprimieren 75,1 % der Zellen das H-2K^{k}-Antigen, was einer sehr hohen Transfektionseffizienz entspricht.

### Beispiel 5

### Transfektion von menschlichen Endothelzellen aus der Nabelschnurvene (HUVEC)

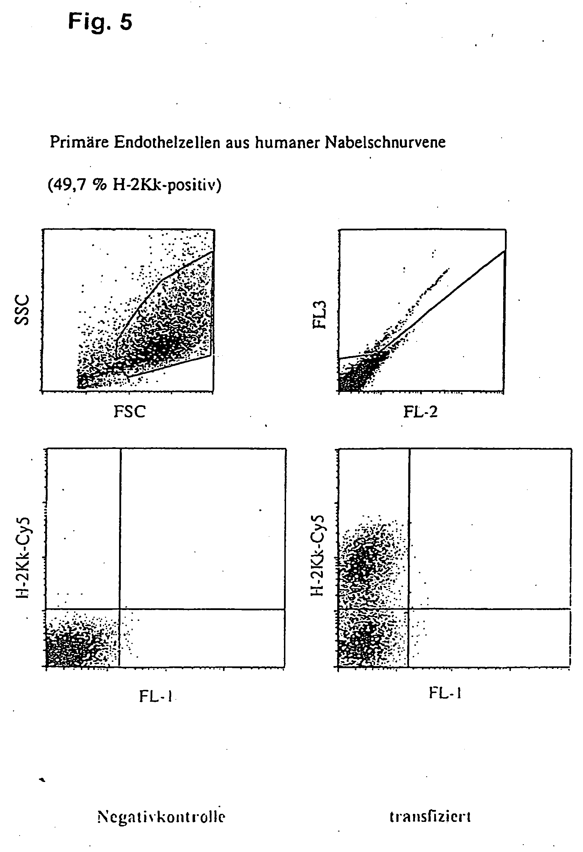

Endothelzellen aus humaner Nabelschnur (1 x 10⁶ Zellen) wurden mit 5 µg H-2K^{k}-Expressionsvektor-DNA in in einem Puffer mit hoher Pufferkapazität (67 mM x pH⁻¹) und hoher lonenstärke (378 mM) bei Raumtemperatur in eine Küvette mit 2 mm Elektrodenabstand gegeben und durch einen Puls von 1000 V und 100 µs Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 5 h Inkubation wurden die Zellen mit einem Cy5-gekoppeltem anti-H-2K^{k}-Antikörper inkubiert und mit einem Durchfluß-Zytometer (FACScalibur, Becton Dickinson) analysiert. Durch Färbung mit Propidiumjodid wurde die Anzahl toter Zellen bestimmt. Wie in Figur 5 gezeigt, exprimieren 49,7 % der lebenden Zellen das H-2K^{k}-Antigen, was einer hohen Transfektionseffizienz entspricht.

### Beispiel 6

### Transfektion der humanen Zellinie K562

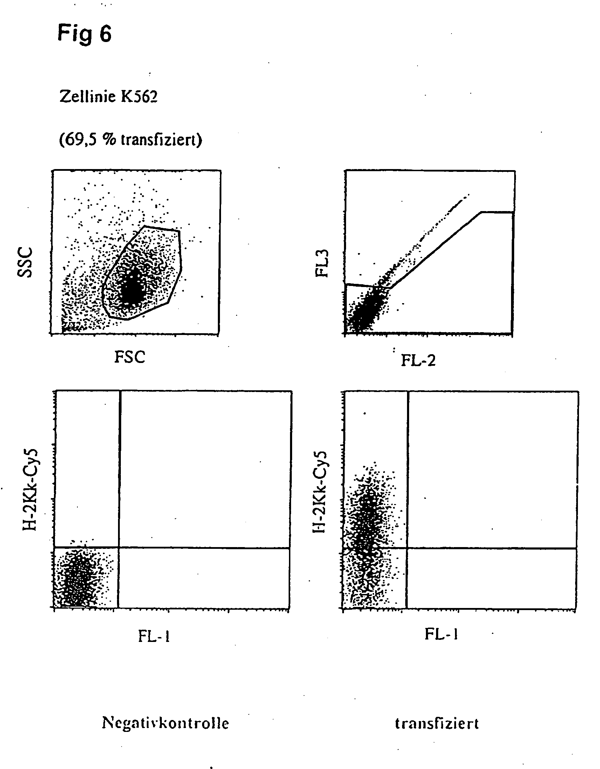

K562-Zellen (1 x 10⁶ Zellen) wurden mit 5 µg H-2K^{k}-Expressionsvektor-DNA in einem Puffer mit hoher Pufferkapazität (24 mM x pH⁻¹) und hoher Ionenstärke (254 mM) bei Raumtemperatur in eine Küvette mit 2 mm Elektrodenabstand gegeben und durch einen Puls von 1000V und 100 µs Dauer, gefolgt von einem Stromfluss mit einer Stromdichte von 8 A cm⁻² und 10 ms Dauer transfiziert. Unmittelbar danach wurden die Zellen mit 400 µl Kulturmedium aus der Küvette gespült, für 10 Minuten bei 37°C inkubiert und dann in eine Kulturschale mit vorgewärmtem Medium überführt. Nach 4 h Inkubation wurden die Zellen mit einem Cy5-gekoppeltem anti-H-2K^{k}-Antikörper inkubiert und mit einem Durchfluß-Zytometer (FACScalibur, Becton Dickinson) analysiert. Durch Färbung mit Propidiumjodid wurde die Anzahl toter Zellen bestimmt. Wie in Figur 6 gezeigt, exprimieren 69,5 % der Zellen das H-2K^{k}-Antigen, was ebenfalls einer hohen Transfektionseffizienz entspricht.

### Beispiel 7

### Transfektionseffizienz und durchschnittliche Fluoreszenzintensität Cycle3-GFP-transfizierter CHO-Zellen

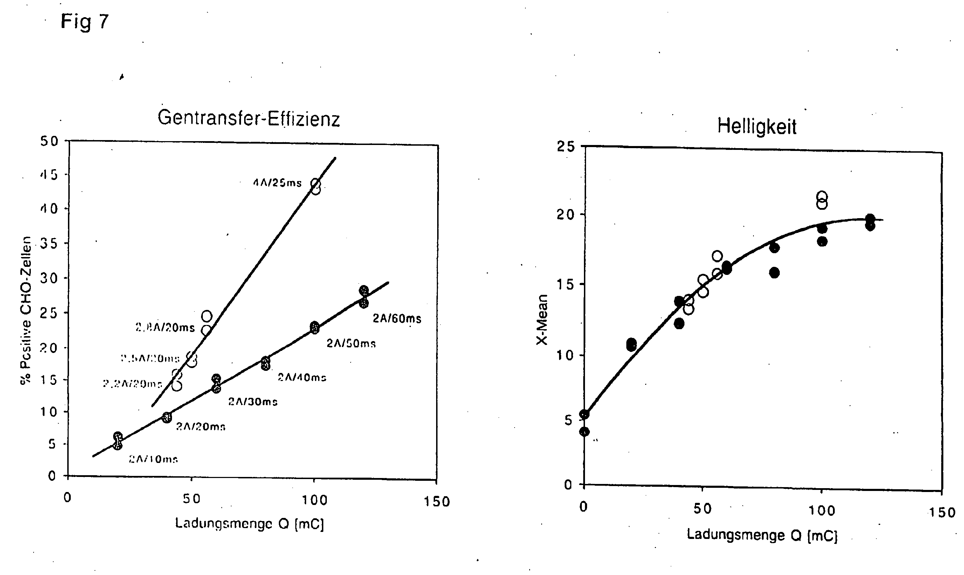

Um die Transfektionseffizienz und die mittlere Fluoreszenzintensität transfizierter Zellen in Abhängigkeit von der geflossenen Ladungsmenge im zweiten Puls zu untersuchten, wurden je 7 x 10⁵ CHO-Zellen mit 5 µg Cycle3-GFP-Vektor-DNA in Elektroporationspuffer in eine Küvette mit 2 mm Elektrodenabstand gegeben und durch einen Puls von 1000 V und 10 µs und anschließende 2. Pulse transfiziert, die sich durch Variation von Stromstärke bzw. Stromdichte und Pulszeit unterscheiden. Nach 5 Stunden Kultivierung wurden die Zellen durchflusszytometrisch analysiert. Figur 7 zeigt die ermittelte Transfektionseffizienz in Abhängigkeit des Integrals von Strom über die Pulszeit (Ladungsmenge Q). Es zeigt sich, dass die Transfektionseffizienz mit Erhöhung der Stromstärke gesteigert werden kann (offene Kreise). Eine Erhöhung der Pulszeit bei gleichbleibender Stromstärke hingegen resultiert in keiner erheblichen Steigerung der Effizienz (geschlossene Kreise). Die Fluoreszenzintensität (Helligkeit) der transfizierten Zellen steigt mit einer Erhöhung der Ladungsmenge Q, wobei eine Sättigung bei hohem Q erreicht wird. Es zeigen sich keine großen Unterschiede, ob die Q-Erhöhung durch Steigerung der Stromstärke (offene Kreise) oder Verlängerung der Pulse (geschlossene Kreise) erzielt wurde.

### Beispiel 8

### Transfektionseffizienz und durchschnittliche Fluoreszenzintensität Cycle3-GFP-transfizierter Jurkat-Zellen

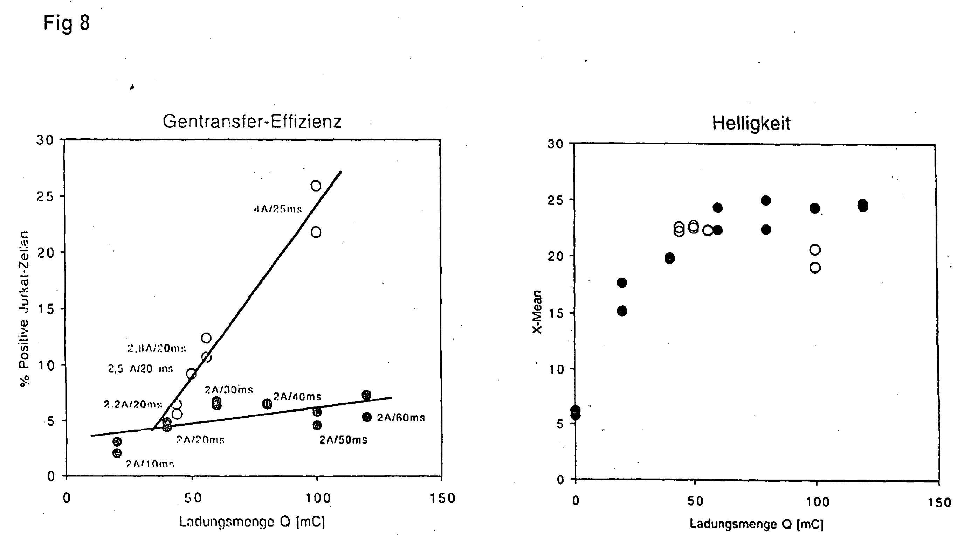

Um die Transfektionseffizienz und die mittlere Fluoreszenzintensität transfizierter Zellen in Abhängigkeit von der geflossenen Ladungsmenge im zweiten Puls zu untersuchten, wurden je 4,5 x 10⁵ Jurkat-Zellen mit 5 µg Cycle3-GFP-Vektor-DNA in Elektroporationspuffer in eine Küvette mit 2 mm Elektrodenabstand gegeben und durch einen Puls von 1000V und 10 µs und anschließende 2. Pulse transfiziert, die sich durch Variation von Stromstärke bzw. Stromdichte und Pulszeit unterscheiden. Nach 5 Stunden Kultivierung wurden die Zellen durchflusszytometrisch analysiert. Figur 8 zeigt die ermittelte Transfektionseffizienz in Abhängigkeit des Integrals von Strom über die Pulszeit (Ladungsmenge Q). Wie bei Verwendung von CHO-Zellen zeigt sich, dass die Transfektionseffizienz mit Erhöhung der Stromstärke gesteigert werden kann (offene Kreise). Eine Erhöhung der Pulszeit bei gleichbleibender Stromstärke hingegen resultiert in keiner erheblichen Steigerung der Effizienz (geschlossene Kreise). Die Fluoreszenzintensität (Helligkeit) der transfizierten Zellen steigt mit einer Erhöhung der Ladungsmenge Q, wobei eine Sättigung bei hohem Q erreicht wird. Es zeigen sich keine großen Unterschiede, ob die Q-Erhöhung durch Steigerung der Stromstärke (offene Kreise) oder Verlängerung der Pulse (geschlossene Kreise) erzielt wurde.

### Beispiel 9

### Transfektionseffizienz und durchschnittliche Fluoreszenzintensität H-2K^{k}-transfizierter Jurkat-Zellen

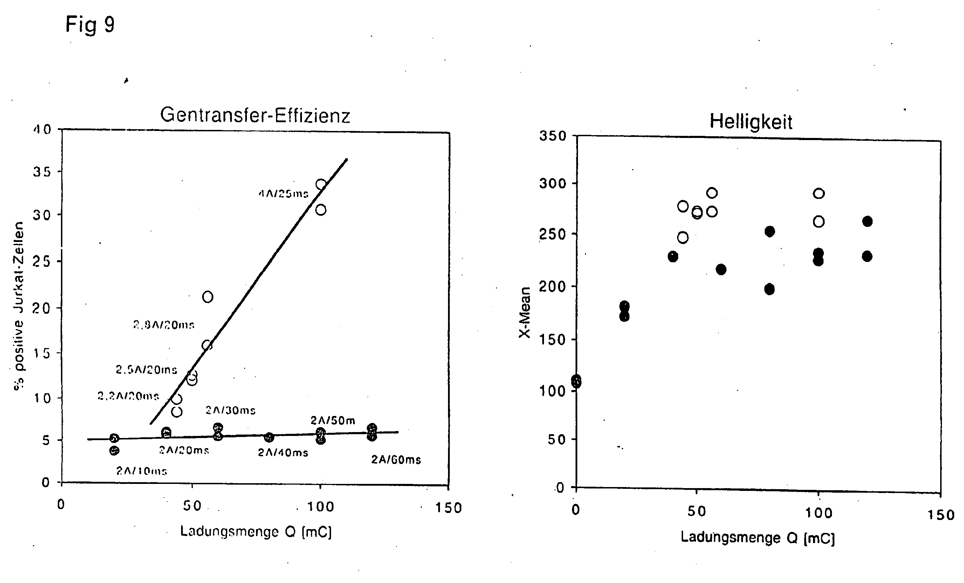

Um die Transfektionseffizienz und die mittlere Fluoreszenzintensität transfizierter Zellen in Abhängigkeit von der geflossenen Ladungsmenge im zweiten Puls zu untersuchten, wurden je 1 x 10⁶ Jurkat-Zellen mit 2 µg H2K^{k}-Expressionsvektor-DNA in Elektroporationspuffer in eine Küvette mit 2 mm Elektrodenabstand gegeben und durch einen Puls von 1000 V und 10 µs und anschließende 2. Pulse transfiziert, die sich durch Variation von Stromstärke bzw. Stromdichte und Pulszeit unterscheiden. Nach 3,5 Stunden Kultivierung wurden die Zellen mit Cy5-gekoppeltem anti-H2K^{k} inkubiert und durchflusszytometrisch analysiert. Figur 9 zeigt die ermittelte Transfektionseffizienz in Abhängigkeit des Integrals von Strom über die Pulszeit (Ladungsmenge Q). Es zeigt sich, dass die Transfektionseffizienz mit Erhöhung der Stromstärke gesteigert werden kann (offene Kreise). Eine Erhöhung der Pulszeit bei gleichbleibender Stromstärke hingegen resultiert in keiner erheblichen Steigerung der Effizienz (geschlossene Kreise). Die Fluoreszenzintensität (Helligkeit) der transfizierten Zellen steigt mit einer Erhöhung der Ladungsmenge Q, wobei eine Sättigung bei hohem Q erreicht wird. Es zeigen sich keine großen Unterschiede, ob die Q-Erhöhung durch Steigerung der Stromstärke (offene Kreise) oder Verlängerung der Pulse (geschlossene Kreise) erzielt wurde.

Die Erfindung wird weiterhin durch die folgenden Figuren erläutert.

Es zeigt
- Figur 1: eine Transfektion von zytotoxischen T-Zellen aus menschlichem Blut
- Figur 2: eine Transfektion von pluripotenten Vorläuferzellen des menschlichen Bluts
- Figur 3: eine Transfektion von menschlichen neonatalen dermalen Fibroblasten
- Figur 4: eine Transfektion von menschlichen neonatalen dermalen Melanozyten
- Figur 5: eine Transfektion menschlicher Endothelzellen aus der Nabelschnurvene
- Figur 6: eine Transfektion der Zelllinie K562 (Analyse 4h nach Transfektion)
- Figur 7: eine Untersuchung der Effizienz der Transfektion in Abhängigkeit von Stromstärke, lmpulszeit und Ladungsmenge und der Intensität der Expression in Abhängigkeit von der Ladungsmenge, Versuch mit der Zelllinie CHO
- Figur 8: eine Untersuchung der Effizienz der Transfektion in Abhängigkeit von Stromstärke, lmpulszeit und Ladungsmenge und der Intensität der Expression in Abhängigkeit von der Ladungsmenge, Versuch mit der Zelllinie Jurkat
- Figur 9: eine Untersuchung der Effizienz der Transfektion in Abhängigkeit von Stromstärke, lmpulszeit und Ladungsmenge und der Intensität der Expression in Abhängigkeit von der Ladungsmenge, Versuch mit der Zelllinie Jurkat und dem Oberflächenmarkerprotein H-2K^{k}.
- Figur 10: ein Blockschaltbild einer Elektroporatorschaltung,
- Figur 11: ein Flussdiagramm zur Erläuterung des Ablaufs der lmpulsabgabevorgänge.

**Figur 1** zeigt die durchfluß-zytometrische Analyse von PBMC, die mit dem H-2K^{k}-Expressionsvektor transfiziert worden sind. Die Zellen wurden nacheinander mit Digoxygenin-gekoppeltem anti-H-2K^{k}-Antikörper und Cy5-gekoppeltem anti-Digoxygenin-Antikörper, sowie mit einem PerCP-gekoppeltem anti-CD8-Antikörper zur Identifizierung humaner zytotoxischer T-Zellen inkubiert und mit einem Durchfluß-Zytometer (FACScalibur, Becton Dickinson) analysiert. (FL-2, FL-3 = Fluoreszenzkanal 2, 3; SSC = sideward scatter, FSC = forward scatter, PerCP = Peridinin chlorophyll protein, CD = cluster of differentiation)

**Figur 2** zeigt die durchfluß-zytometrische Analyse von aus PBMC angereicherten CD34-positiven Stammzellen, die mit dem H-2K^{k}-Expressionsvektor transfiziert worden sind. Die Zellen wurden nacheinander mit Phycoerythrin-gekoppeltem anti-H-2K^{k}-Antikörper, sowie mit einem APCgekoppeltem anti-CD34-Antikörper zur Identifizierung von humanen CD34-positiven hämatopoietischen Stammzellen inkubiert und mit einem Durchfluß-Zytometer (FACScalibur, Becton Dickinson) analysiert. (FL-1, FL-3 = Fluoreszenzkanal 1, 3; SSC = sideward scatter, FSC = forward scatter, PE = Phycoerythrin, APC = Allophycocyanin, CD = cluster of differentiation)

**Figur 3** zeigt die durchfluß-zytometrische Analyse von humanen neonatalen dermalen Fibroblasten (NHDF-Neo), die mit dem H-2K^{k}-Expressionsvektor transfiziert worden sind. Die Zellen wurden mit einem Cy5-gekoppeltem anti-H-2K^{k} inkubiert und mit einem Durchfluß-Zytometer (FACScalibur, Becton Dickinson) analysiert. (FL-1, FL-2, FL-3 = Fluoreszenzkanal 1, 2, 3; SSC = sideward scatter, FSC = forward scatter)

**Figur 4** zeigt die durchfluß-zytometrische Analyse von humanen neonatalen Melanozyten (NHEM-Neo), die mit dem H-2K^{k}-Expressionsvektor transfiziert worden sind. Die Zellen wurden mit einem Cy5-gekoppeltem anti-H-2K^{k} inkubiert und mit einem Durchfluß-Zytometer (FACScalibur, Becton Dickinson) analysiert. (FL-1, FL-2, FL-3 = Fluoreszenzkanal 1, 2, 3; SSC = sideward scatter, FSC = forward scatter)

**Figur 5** zeigt die durchfluß-zytometrische Analyse von Endothelzellen aus humaner Nabelschnur (HUVEC), die mit dem H-2K^{k}-Expressionsvektor transfiziert worden sind. Die Zellen wurden mit einem Cy5-gekoppeltem anti-H-2K^{k} inkubiert und mit einem Durchfluß-Zytometer (FACScalibur, Becton Dickinson) analysiert. (FL-1, FL-2, FL-3 = Fluoreszenzkanal 1, 2, 3; SSC = sideward scatter, FSC = forward scatter)

**Figur 6** zeigt die durchfluß-zytometrische Analyse der humanen Zellinie K562, die mit dem H-2K^{k}-Expressionsvektor transfiziert worden sind. Die Zellen wurden mit einem Cy5-gekoppeltem anti-H-2K^{k} -Antikörper inkubiert und mit einem Durchfluß-Zytometer (FACScalibur, Becton Dickinson) analysiert. (FL-1, FL-2, FL-3 = Fluoreszenzkanal 1, 2, 3; SSC = sideward scatter, FSC = forward scatter)

**Figur 7** zeigt eine graphische Darstellung der Transfektionseffizienz von CHO-Zellen und der durchschnittlichen Fluoreszenzintensität (Helligkeit) der positiven Zellen in Abhängigkeit von der geflossenen Ladungsmenge Q. Die CHO-Zellen wurden mit dem Cycle3-GFP-Expressionsvektor transfiziert und nach fünf Stunden mit einem Durchfluss-Zytometer (FACScalibur, Becton Dickinson) analysiert. Geschlossene Kreise entsprechen einer schrittweisen Steigerung der Pulszeit bei gleichbleibender Stromstärke (2 A) bzw. Stromdichte (4 A cm⁻²), offene Kreise einer Steigerung der Stromstärke.

**Figur 8** zeigt eine graphische Darstellung der Transfektionseffizienz von Jurkat-Zellen und der durchschnittlichen Fluoreszenzintensität (Helligkeit) der positiven Zellen in Abhängigkeit von der geflossenen Ladungsmenge Q. Die Jurkat-Zellen wurden mit dem Cycle3-GFP-Expressionsvektor transfiziert und nach fünf Stunden mit einem Durchfluss-Zytometer (FACScalibur, Becton Dickinson) analysiert. Geschlossene Kreise entsprechen einer schrittweisen Steigerung der Pulszeit bei gleichbleibender Stromstärke (2 A) bzw. Stromdichte (4 A cm⁻²), offene Kreise einer Steigerung der Stromstärke.

**Figur 9** zeigt eine graphische Darstellung der Transfektionseffizienz von Jurkat-Zellen und der durchschnittlichen Fluoreszenzintensität (Helligkeit) der positiven Zellen in Abhängigkeit von der geflossenen Ladungsmenge Q. Die Jurkat-Zellen wurden mit dem H-2K^{k}-Expressionsvektor transfiziert und nach dreieinhalb Stunden mit einem Cy5-gekoppelten anti-H-2K^{k} inkubiert und mit einem Durchfluss-Zytometer (FACScalibur, Becton Dickinson) analysiert. Geschlossene Kreise entsprechen einer schrittweisen Steigerung der Pulszeit bei gleichbleibender Stromstärke (2 A) bzw. Stromdichte (4 A cm⁻²), offene Kreise einer Steigerung der Stromstärke.

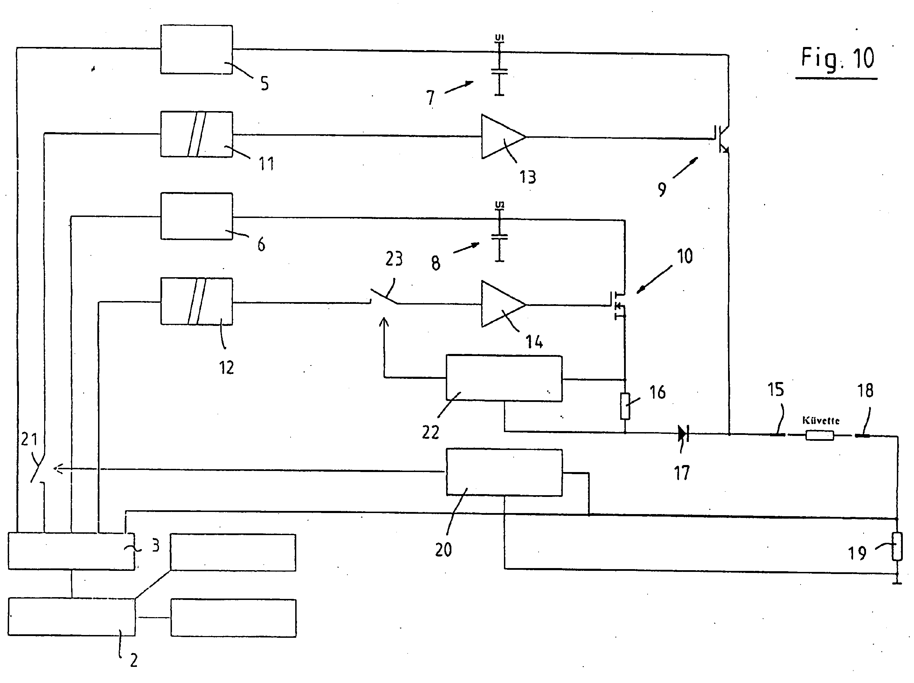

**Figur 10** zeigt ein Blockschaltbild des Elektroporators 1 zur Durchführung des erfindungsgemäßen Verfahrens mit den notwendigen Einzelbestandteilen. Es handelt sich um eine Einstellungseinheit 2, eine Steuerungseinheit 3 mit angeschlossener Spannungsversorgungseinheit 4 sowie zumindest zwei HV-Netzteilen 5,6 mit nachgeschalteter Speichereinrichtung 7,8 und zwei zur lmpulsabgabe vorgesehenen Leistungshalbleitern 9,10. Die Leistungshalbleiter 9,10 werden über eine Potentialtrennstufe 11,12 durch die Steuerungseinheit 3 über einen HV-Schalter 13 und eine Regelungseinheit 14 angesteuert. Die Speichereinrichtungen 7,8 sind mit den Eingängen der Leistungshalbleiter 9,10 unmittelbar verbunden, wobei die Speichereinrichtungen 7,8 aus einem oder mehreren Kondensatoren je nach verwendeter Feldstärke und Impulsdauer bestehen können. Der Leistungshalbleiter 9 kann beispielsweise aus einem IGBT und der Leistungshalbleiter 10 aus einem MOSFET bestehen. Der Begriff "Leistungshalbleiter" soll jedoch auch sämtliche anderen elektronischen Bauelemente oder Bauelementanordnungen umfassen, mittels derer die im Rahmen der Erfindung zu schaltenden Spannungen und Ströme mit den erforderlichen Schaltzeiten geschaltet werden können. Der Ausgang des IGBT ist unmittelbar mit dem Küvettenanschluss 15 verbunden, während der Ausgang des MOSFET 10 über einen Widerstand 16 und eine Diode 17 mit dem Küvettenanschluss 15 verbunden ist, sodass kein Impuls über den zweiten Leistungshalbleiter 10 zurückfließen kann, falls beide Leistungshalbleiter 9, 10 gleichzeitig angesteuert sind. Die Diode 17 ist hierzu kathodenseitig mit dem Küvettenanschluss 15 verbunden. Der zweite Küvettenanschluss 18 ist über einen Widerstand 19 mit Masse verbunden. Bei dem Widerstand 19 handelt es sich um ein Mess-Shunt um den Spannungsabfall zu messen und einer Überstromschaltstufe 20 zuzuführen. Die Überstromschaltstufe 20 kann über einen Schalter 21 eine Unterbrechung der lmpulsabgabe über die Potentialtrennstufe 11 und den HV-Schalter 13 vornehmen, während eine zweite Überstromschaltstufe 22 über einen Schalter 23 eine Ansteuerung der Regelungseinheit 14 für den MOSFET 10 unterbricht. Der Überstromschaltstufe 22 wird die über den Widerstand 16 anliegende Spannung zugeleitet, um im Fall einer Überschreitung des Maximalstroms eine Stromabschaltung herbeizuführen. Dadurch, dass der Widerstand 16 unmittelbar im Hochspannungskreis liegend angeordnet ist, befindet sich der Schalter 23 hinter der Potentialtrennstufe 12, sodass keine Hochspannungsimpulse in die Steuerungseinheit 3 gelangen können und das Bedienungspersonal nicht gefährdet ist. Im Fall der Überstromschaltstufe 20 liegt der niederohmige Messwiderstand 19 hinter den Küvettenanschlüssen 15,18 und ist gegen Masse verschaltet, sodass die Übertragung von Hochspannungsimpulsen ausgeschlossen werden kann. Je nach Verwendungszweck des Elektroporators 1 können ein oder mehrere Hochspannungsnetzteile 5,6 mit zugehörigen Speichereinrichtungen 7,8 sowie notwendigen Potentialtrennstufen 11,12 und HV-Schalter 13 bzw. Regelungseinheit 14 zur Ansteuerung der Leistungshalbleiter 9,10 eingesetzt werden. Die Speichereinrichtungen 7,8 sind mit einem oder mehreren Kondensatoren der notwendigen Kapazität und Durchschlagsspannung ausgestattet, sodass eine entsprechend hohe Ladungsmenge gespeichert und auf dem Küvettenanschluss 15 übertragen werden kann.

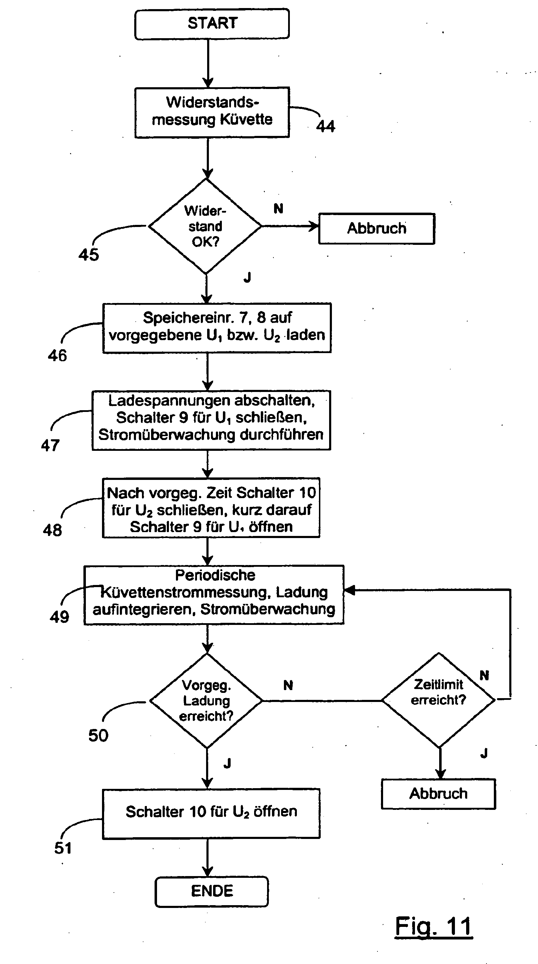

**Figur 11** zeigt ein schematisches Flussdiagramm des Betriebsablaufs eines von der Steuerungseinheit 3 (vgl. Figur 10) kontrollierten lmpulsabgabevorgangs gemäß einer bevorzugten Ausführungsform der Erfindung. Zunächst werden die erforderlichen lmpulsparameter manuell oder durch Auslesen einer Speicherkarte vorgegeben (nicht dargestellt). Nach dem Start des Vorganges (z.B. durch Betätigung einer entsprechenden Auslösetaste) wird zunächst der ohmsche Widerstand der Küvette durch kurzzeitiges Anlegen einer Niederspannung (z.B. 12 V) an die Küvettenanschlüsse 15,18 und eine anschließende Strommessung (z.B. für 2 ms) in Schritt 44 gemessen. Im Rahmen der Abfrage 45 wird überprüft, ob dieser Widerstand innerhalb eines vorgegebenen Fensters liegt. Andernfalls wird der weitere Vorgang abgebrochen. Der gemessene Widerstandswert wird für die Berechnung der Ladespannung U₂ in der vorliegenden Ausführungsform der Erfindung nicht weiter herangezogen. Liegt der Widerstand ordnungsgemäß innerhalb des vorgegebenen Fensters, so werden die Speichereinrichtungen 7, 8 in Schritt 46 auf die vorgegebenen Spannungen U₁ und U₂ aufgeladen. Sind die gewünschten Ladespannungen erreicht, so wird die Aufladung durch die HV-Netzteile 5, 6 abgeschaltet. Während der nachfolgenden lmpulsabgabe erfolgt keine Nachladung der Speichereinrichtungen. Die Impulsabgabe für den Hochspannungsimpuls beginnt sodann in Schritt 47 durch Schließen des Halbleiterschalters 9. Dadurch fließt ein relativ hoher Strom durch die Küvette. Ein zu steiler Anstieg des Stroms wird durch eine Überstromabschaltstufe 20 erkannt und führt aus Sicherheitsgründen zu einem sofortigen Öffnen des Schalters 9 und einem Abbruch der Routine. In der vorliegenden Ausführungsform wird der Hochspannungsimpuls nach einer vorgegebenen Zeitdauer von einigen Mikrosekunden beendet, worauf sich unmittelbar und ohne Unterbrechung der zweite Impuls anschließt. Hierzu wird in Schritt 48 der zweite Halbleiterschalter 10 bereits kurze Zeit vor Öffnen des ersten Halbleiterschalters 9 geschlossen, so dass sich ein unterbrechnungsfreier Übergang zwischen den beiden Impulsen ergibt. In der kurzen Zeitspanne, in der beide Hochspannungsschalter 9, 10 gleichzeitig geschlossen sind, verhindert die Diode 17, dass eine evtl. höhere Spannung aus der Speichereinrichtung 7 in die Speichereinrichtung 8 fließen kann. Der Halbleiterschalter 10 bleibt anschließend solange geöffnet (sofern der Maximalstrom durch eine Überstromabschaltstufe 22 nicht überschritten wird), bis die vorgegebene Ladung Q durch die Küvette geflossen ist. Dazu wird in Schritt 49 in vorgegebenen Zeitintervallen (z.B. 1 ms) der durch die Küvette fließende Strom gemessen und aufintegriert. Sobald die vorgegebene Soll-Ladung erreicht ist (vgl. Abfrage 50), wird der Schalter 10 geöffnet und die Routine beendet. Die Kapazität der Speichereinrichtung 8 ist so gewählt, dass die Spannung während der Dauer des zweiten Impulses allmählich bzw. langsam abfällt. Falls aufgrund einer Störung die vorgegebene Soll-Ladung auch bei nahezu vollständig entladener Speichereinrichtung immer noch nicht erreicht ist, so wird nach Überschreiten eines entsprechend gewählten Zeitlimits der Vorgang ebenfalls abgebrochen.

### Bezugszeichenliste

- 1: Elektroporator
- 2: Einstellungseinheit
- 3: Steuerungseinheit
- 4: Spannungsversorgungseinheit
- 5: HV-Netzteil
- 6: HV-Netzteil
- 7: Speichereinrichtung
- 8: Speichereinrichtung
- 9: Leistungshalbleiter
- 10: Leistungshalbleiter
- 11: Potentialtrennstufe
- 12: Potentialtrennstufe
- 13: HV-Schalter
- 14: Regelungseinheit
- 15: Küvettenanschluss
- 16: Widerstand
- 17: Diode
- 18: Küvettenanschluss
- 19: Widerstand
- 20: Überstromabschaltstufe
- 21: Schalter
- 22: Überstromabschaltstufe
- 23: Schalter
- 30: Tastschalter
- 31: Anzeigelement
- 32: LED
- 33: Anschlussstecker
- 34: Kartenleser
- 35: Umschalter
- 36: Transformator
- 37: Gleichrichter
- 38: SpannungsRegeler
- 39: Regelstufe
- 40: Transformatorstufe
- 41: Lötpad
- 42: Lötpad
- 43: lmpulskontrollstufe
- 44: bis 51 Schritte

## Patentansprüche

1. Verfahren zur Einbringung von Nukleinsäuren, Peptiden, Proteinen und/oder anderen biologisch aktiven Molekülen in den Zellkern eukaryontischer Zellen mittels elektrischen Stroms, wobei zumindest ein Spannungsimpuls auf die Zellen übertragen wird, **dadurch gekennzeichnet, dass** die durch den Spannungsimpuls abgegebene Ladungsmenge in zumindest einem wählbaren Zeitintervall gemessen wird, wobei ein Vergleich einer voreingestellten Soll-Ladungsmenge mit der abgegebenen Ist-Ladungsmenge durchgeführt wird, und dass bei Erreichen oder Überschreiten der Soll-Ladungsmenge der Spannungsimpuls abgebrochen wird.

2. Verfahren nach Anspruch 1, **dadurch gekennzeichnet, dass** zunächst ein erster Impuls mit der Spannung U₁ auf die Zellen übertragen wird und dass anschließend ohne Unterbrechung zumindest ein zweiter Impuls mit der Spannung U₂ ebenfalls auf die Zellen übertragen wird.

3. Verfahren nach Anspruch 1 oder 2, **dadurch gekennzeichnet, dass** die Bestimmung der abgegebenen Ladungsmenge aus der Differenz zwischen der ursprünglichen Ladung einer entsprechenden Speichereinrichtung (7,8) und der restlichen Ladung erfolgt.

4. Verfahren nach einem oder mehreren der Ansprüche 1 - 3, **dadurch gekennzeichnet, dass** ein erster Impuls mit einer Feldstärke von 2 - 10 kV/cm und mit einer Dauer von 10 - 100 µs und einer Stromdichte von mindestens 2 A cm⁻² und anschließend ohne Unterbrechung ein zweiter Impuls mit einer Stromdichte von 2 - 14 A cm⁻² und einer Dauer von maximal 100 ms auf die Zellen übertragen wird.

5. Verfahren nach einem oder mehreren der Ansprüche 1 - 4, **dadurch gekennzeichnet, dass** das Zeitintervall zur Bestimmung der abgegebenen Ladung zeitgleich mit der Abgabe der Ladung des ersten und/oder vorzugsweise eines zweiten oder jeden weiteren Impulses festgelegt wird.

6. Verfahren nach einem oder mehreren der Ansprüche 1 - 5, **dadurch gekennzeichnet, dass** die Einschaltdauer (T₂) des zweiten Impulses durch einen Vergleich der Soll-Ladungsmenge mit der bis zum Messzeitpunkt abgegebenen Ist-Ladungsmenge festgelegt wird und mit Erreichen der Soll-Ladungsmenge beendet wird.

7. Verfahren nach einem oder mehreren der Ansprüche 1 - 6, **dadurch gekennzeichnet, dass** zur Bestimmung der Ist-Ladungsmenge ein Messzyklus von 1 msec gewählt wird, wobei während der Zeit (T₂) die Kondensatorspannung exponentiell abfällt und der Impuls bei Erreichen der festgelegten Ladungsmenge (Q₂) abgebrochen wird.

8. Verfahren nach einem oder mehreren der Ansprüche 1 - 7, **dadurch gekennzeichnet, dass** nach zumindest einem vorbestimmten Zeitintervall nach Auslösen eines ersten und/oder zweiten Impulses der fließende Strom gemessen wird und bei Über- oder Unterschreiten eines Sollwertes die Dauer des Pulses nachgeregelt wird, um die abgegebene Ladungsmenge konstant zu halten.

9. Verfahren nach einem oder mehreren der Ansprüche 1 - 7, **dadurch gekennzeichnet, dass** nach zumindest einem vorbestimmten Zeitintervall nach Auslösen eines ersten und/oder zweiten Impulses der fließende Strom gemessen wird und bei Über- oder Unterschreiten eines Sollwertes eine Fehlermeldung erfolgt.

10. Verfahren nach einem oder mehreren der Ansprüche 1 - 7, **dadurch gekennzeichnet, dass** nach zumindest einem vorbestimmten Zeitintervall nach Auslösen eines ersten und/oder zweiten Impulses der fließende Strom gemessen wird und bei Über- oder Unterschreiten des Sollwertes eine Nachregelung an den Sollwert erfolgt.

11. Verfahren nach einem oder mehreren der Ansprüche 1 - 10, **dadurch gekennzeichnet, dass** vorselektierte Einstellungsparameter der Impulse (U₁, T₁, I₂, T₂, K₂) manuell oder über die Eingabe eines Codes eingegeben werden.

12. Verfahren nach einem oder mehreren der Ansprüche 1 - 11, **dadurch gekennzeichnet, dass** eine Überstromabschaltung für den ersten und den zweiten Impuls durchgeführt wird.

13. Verfahren nach einem oder mehreren der Ansprüche 1 - 12, **dadurch gekennzeichnet, dass** der für die Berechnung von U₂ verwendete Widerstand R einer Küvette durch eine Widerstandsmessung vor Ansteuerung eines Leistungshalbleiters (9, 19) ermittelt wird.

14. Verfahren nach einem oder mehreren der Ansprüche 1 - 12, **dadurch gekennzeichnet, dass** der für die Berechnung von U₂ verwendete Widerstand R der Küvette vorgegeben wird.

15. Verfahren nach einem oder mehreren der Ansprüche 1 - 14, **dadurch gekennzeichnet, dass** vorselektierte Einstellungsparameter der Impulse (U₁, T₁, I₂, T₂, K₂, R) über eine Speicherkarte eingelesen werden.

## Claims

1. A method for introducing nucleic acids, peptides, proteins and/or other biologically active molecules into the cell nucleus of eukaryotic cells by means of electric current, wherein at least one voltage pulse is supplied to the cells, **characterized in that** the quantity of charge supplied by the voltage pulse is measured in at least one selectable time interval, wherein a preset desired quantity of charge is compared with the actually supplied quantity of charge, and wherein the voltage pulse is terminated on reaching or exceeding the desired quantity of charge.

2. The method according to Claim 1, **characterized in that** a first pulse with the capacitor voltage (U₁) is supplied to the cells and subsequently without interruption at least one second pulse with the capacitor voltage (U₂) is also supplied to the cells.

3. The method according to Claim 1 or 2, **characterized in that** the supplied quantity of charge is determined from the difference between the original charge of a corresponding storage device (7,8) and the residual charge.

4. The method according to any of the Claims 1 to 3, **characterized in that** a first pulse having a field strength of 2 - 10 kV/cm, a duration of 10 - 100 µs and a current density of at least 2 A. cm⁻² is applied to the cells, and subsequently without interruption a second pulse having a current density of 2 - 14 A·cm⁻² and a maximum duration of 100 ms is also supplied to the cells.

5. The method according to any of the Claims 1 to 4, **characterized in that** the time interval for determining the supplied charge is specified simultaneously with the supply of the charge of a first and/or preferably a second or each further pulse.

6. The method according to any of the Claims 1 to 5, **characterized in that** the switch-on time (T₂) of the second pulse is specified by comparing the desired quantity of charge with the actual quantity of charge supplied by the measurement time and terminated when the desired quantity of charge is reached.

7. The method according to any of the Claims 1 to 6, **characterized in that** in order to determine the actual quantity of charge a measuring cycle of 1 msec is selected, wherein during the time (T₂) the capacitor voltage decreases exponentially and the pulse is terminated on reaching the specified quantity of charge (Q₂).

8. The method according to any of the Claims 1 to 7, **characterized in that** after at least one pre-determined time interval after triggering one first and/or second pulse the flowing current is measured and, if said current exceeds or falls below a desired value, the duration of the pulse is readjusted in order to keep the supplied quantity of charge constant.

9. The method according to any of the Claims 1 to 7, **characterized in that** after at least one pre-determined time interval after triggering one first and/or second pulse the flowing current is measured and, if said current exceeds or falls below a desired value, an error message is given.

10. The method according to any of the Claims 1 to 7, **characterized in that** after at least one pre-determined time interval after triggering one first and/or second pulse the flowing current is measured and, if said current exceeds or falls below the desired value, the desired value is readjusted.

11. The method according to any of the Claims 1 to 10, **characterized in that** the pre-selected setting parameters of the pulse (U₁, T₁, I₂, T₂, K₂) are inputted manually or by entering a code.

12. The method according to any of the Claims 1 to 11, **characterized in that** an overcurrent cutoff for the first and second pulse is accomplished.

13. The method according to any of the Claims 1 to 12, **characterized in that** the resistance R of the cuvette used to calculate (U₂) is determined by a resistance measurement before triggering a power semiconductor (9,19).

14. The method according to any of the Claims 1 to 12, **characterized in that** the resistance R of the cuvette used to calculate (U₂) is predetermined.

15. The method according to any of the Claims 1 to 14, **characterized in that** the pre-selected setting parameters of the pulse (U₁, T₁, I₂, T₂, K₂, R) are read in via a memory card.

## Revendications

1. Procédé pour l'introduction d'acides nucléiques, de peptides, de protéines et/ou d'autres molécules actives au plan biologique dans le noyau de cellules eucaryontiques au moyen du courant électrique, au moins une impulsion de tension étant transmise aux cellules, **caractérisé en ce que** la quantité de charge délivrée par l'impulsion de tension est mesurée dans au moins un intervalle de temps sélectionnable, une comparaison d'une quantité de charge prescrite préréglée avec la quantité de charge réelle délivrée étant effectuée et **en ce que**, lorsque la quantité de charge prescrite est atteinte ou dépassée, l'impulsion de tension est interrompue.

2. Procédé selon la revendication 1, **caractérisé en ce que** d'abord une première impulsion avec la tension U₁ est transmise aux cellules et **en ce qu'**ensuite au moins une seconde impulsion avec la tension U₂ est également transmise sans interruption aux cellules.

3. Procédé selon la revendication 1 ou 2, **caractérisé en ce que** la détermination de la quantité de charge délivrée s'effectue à partir de la différence entre la charge initiale d'un dispositif de stockage (7, 8) approprié et la charge résiduelle.

4. Procédé selon l'une quelconque ou plusieurs des revendications 1 à 3, **caractérisé en ce qu'**une première impulsion d'une intensité de champ 2 à 10 kV/cm et d'une durée de 10 à 100 µs et d'une densité de courant d'au moins 2 A cm⁻² et ensuite une seconde impulsion d'une densité de courant de 2 à 14 A cm⁻² et d'une durée maximale de 100 ms sont transmises aux cellules, la seconde impulsion étant transmise sans interruption.

5. Procédé selon l'une quelconque ou plusieurs des revendications 1 à 4, **caractérisé en ce que** l'intervalle de temps destiné à définir la charge délivrée est fixé en même temps que la délivrance de la charge de la première et/ou de préférence d'une seconde ou de toute autre impulsion.

6. Procédé selon l'une quelconque ou plusieurs des revendications 1 à 5, **caractérisé en ce que** la durée d'enclenchement (T₂) de la seconde impulsion est fixée par une comparaison de la quantité de charge prescrite avec la quantité de charge réelle délivrée jusqu'au moment de mesure et est terminée lorsque la quantité de charge prescrite est atteinte.

7. Procédé selon l'une quelconque ou plusieurs des revendications 1 à 6, **caractérisé en ce que**, pour déterminer la quantité de charge réelle, on choisit un cycle de mesure de 1 msec, la tension de condensateur décroissant de façon exponentielle pendant le temps (T₂) et l'impulsion étant interrompue lorsque la quantité de charge (Q₂) fixée est atteinte.

8. Procédé selon l'une quelconque ou plusieurs des revendications 1 à 7, **caractérisé en ce que**, après au moins un intervalle de temps prédéfini, le courant en circulation est mesuré après le déclenchement d'une première et/ou d'une seconde impulsion et, lorsqu'une valeur prescrite est dépassée ou n'est pas atteinte, la durée de l'impulsion est réajustée afin de maintenir constante la quantité de charge délivrée.

9. Procédé selon l'une quelconque ou plusieurs des revendications 1 à 7, **caractérisé en ce que**, au moins après un intervalle de temps prédéfini et après le déclenchement d'une première et/ou d'une seconde impulsion, le courant en circulation est mesuré et un message d'erreur intervient lorsqu'une valeur prescrite est dépassée ou n'est pas atteinte.

10. Procédé selon l'une quelconque ou plusieurs des revendications 1 à 7, **caractérisé en ce que**, après au moins un intervalle de temps prédéfini et après le déclenchement d'une première et/ou d'une seconde impulsion, le courant en circulation est mesuré et, si la valeur prescrite est dépassée ou n'est pas atteinte, on a un réajustage sur la valeur prescrite.

11. Procédé selon l'une quelconque ou plusieurs des revendications 1 à 10, **caractérisé en ce que** les paramètres de réglage présélectionnés des impulsions (U₁, T₁, I₂, T₂, K₂) sont introduits manuellement ou par l'entrée d'un code.

12. Procédé selon l'une quelconque ou plusieurs des revendications 1 à 11, **caractérisé en ce qu'**une interruption de surintensité est effectuée pour la première et la seconde impulsion.

13. Procédé selon l'une quelconque ou plusieurs des revendications 1 à 12, **caractérisé en ce que** la résistance R, utilisée pour le calcul de U₂, d'une cuvette est déterminée par une mesure de résistance avant l'activation d'un semi-conducteur de puissance (9, 19).

14. Procédé selon l'une quelconque ou plusieurs des revendications 1 à 12, **caractérisé en ce que** la résistance R de la cuvette, qui est utilisée pour le calcul de U₂, est prédéfinie.

15. Procédé selon l'une quelconque ou plusieurs des revendications 1 à 14, **caractérisé en ce que** des paramètres de réglage présélectionnés des impulsions (U₁, T₁, I₂, T₂, K₂, R) sont entrés par une carte mémoire.
